# EUROPEAN PATENT APPLICATION

(11) **EP 0 761 679 A1**
(43) Date of publication of application: **12.03.1997**
(21) Application number: 96306196.5
(22) Date of filing: 27.08.1996
(51) Int. Cl.: C07J 31/00, A61K 31/56

(54) **N-Butylsulfonate esters of estrogens**

(30) Priority: 30.08.1995 US 2959
(71) Applicant: ELI LILLY AND COMPANY, Indianapolis, Indiana 46285 (US)
(72) Inventor: Bryant, Henry Uhlman, Indianapolis, Indiana 46250 (US); Cullinan, George Joseph, Trafalgar, Indiana 46181 (US)
(74) Representative: Hudson, Christopher Mark

(57) **Abstract**

The present invention provides 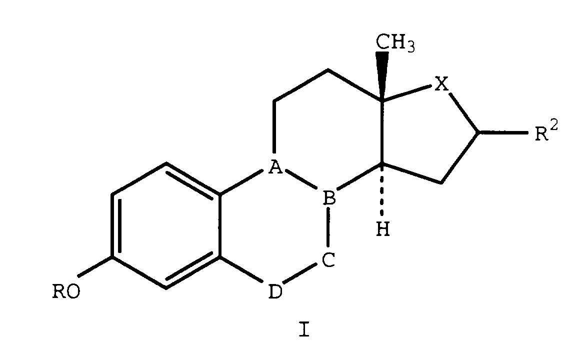 wherein
X is α-CH₂OR¹ when A is α-hydro, A and B together are ethylene, and C and D together are ethylene, β-CH₂OR¹, or -CO-;
R is -H or n-butylsulfonoyl;
R¹ is R provided that at least one of R and R¹ must be n-butylsulfonoyl;
R² is -H, α- or β-hydroxy, or α- or β-n-butylsulfonoyl;
A is 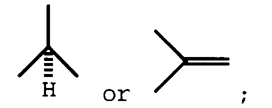
B is 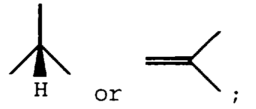
C is -CH₂ or 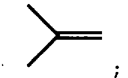 and
D is -CH₂ or 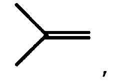 methods of inhibiting estrogen deficient patholigies, and pharmaceutical formulations.

## Description

Estrogens, as a pharmacological class, are among the most widely prescribed drugs in the world. They are useful for treating a wide range of estrogen deficient pathologies which include, for example, lack of birth control, treatment of post-menopausal syndrome, treatment of certain cancers in men such as prostatic carcinoma, acne, hirsutism, dysfunctional uterine bleeding, dysmenorrhea, and atrophic vaginitis. The use of various estrogens, either alone or often in combination with progestins, are generally considered to be safe and effective.

In humans, the endogenous estrogens are predominately 17-β-estradiol, estrone, and estratriol. Although these estrogens are the normal, endogenous hormones, they are not often used via oral administration due to their rapid metabolism to inactive species. The inactivation of these estrogens occurs in the liver followed by rapid excretion as conjugates such as glucuronides or sulfates. Conjugation normally occurs at the phenolic and/or alkyl hydroxyl moiety. In order to circumvent this problem, carboxylic esters such as propionates, undecylates, and valerates have been synthesized. These esters have been shown to be effective in slowing the elimination of the parent estrogen and prolonging the pharmacological action. However, because these esters are unstable to the acid environment of the stomach, these compounds are often unsuitable for oral administration and must be given by parenteral routes.

Other derivatives and preparations of estrogens have been developed for the purpose of circumventing the rapid elimination problem. One solution has been to synthesize a pro-drug form of estradiol which requires metabolic activation in order to release the active moiety. An example of such a pro-drug is 17-α-ethinyl-estradiol. Although this compound is active, orally-available, and long acting, its use is limited to patients which have good liver function for the metabolic conversion. Additionally, 17-α-ethinyl-estradiol requires careful chemical synthesis and preparation, making its preparation relatively expensive in comparison to estrogen derivatives prepared by simpler chemistry. Another solution has been the use of conjugated equine estrogens. These preparations have the advantages of being inexpensive, orally-effective, and reasonably long acting. Conjugated estrogens of this type contain not only the natural human estrogens, but large amounts of equine estrogens. It is not known if the equine estrogens have pharmacological properties which may be harmful in long-term use.

Despite the plethora of estrogens currently available, there exists the need for an orally available, long acting, inexpensive, and safer estrogen derivative which will deliver the naturally occurring human estrogens.

Both alkyl and aryl sulfonate esters have been widely employed in the chemical art for protecting phenolic and alkyl hydroxyl moieties from chemical reaction. Methanesulfonates and phenylsulfonates are commonly used to protect hydroxyl functions in one part of a molecule while other chemical reactions are performed on another part. This protection with sulfonates is common because of the ease of synthesis, resistance to many chemical reactions, and facile removal of the protecting group, usually by base hydrolysis. Among the useful properties of a sulfonate moiety is its resistance to acid hydrolysis. This acid resistance would seem to make the sulfonate moiety ideal as a biological protecting group for transit through the acidic conditions of the stomach. However, sulfonates are not commonly use for this purpose because they are not readily metabolized to their active parent compounds. It would be of great value if an orally-available, sulfonated estrogenic compound were available in which one or more sulfonate moieties served as a biological protecting group for metabolically unstable hydroxyl functions.

The present invention provides n-butylsulfonate esters of the estrogenic compounds of formula I, which are useful for treating estrogen deficient pathologies in mammals. 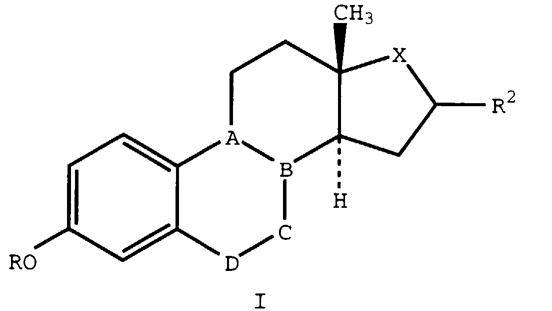 wherein
X is α-CH₂OR¹ when A is α-hydro, A and B together are ethylene, and C and D together are ethylene, β-CH₂OR¹, or -CO-;
R is -H or n-butylsulfonoyl;
R¹ is R provided that at least one of R and R¹ must be n-butylsulfonoyl;
R² is -H, α- or β-hydroxy, or α- or β-n-butylsulfonoyl;
A is 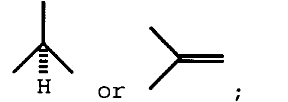
B is 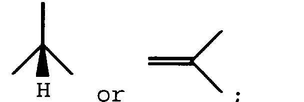
C is -CH₂ or 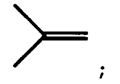 and
D is -CH₂ or

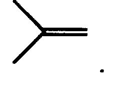

The present invention also provides pharmaceutical formulations of formula I compounds, optionally containing a progestin, as well as methods for treating estrogen dependent pathologies in mammals.

The present invention concerns the discovery that n-butylsulfonate esters of select parent estrogenic compounds have increased bioavailability and potency over their parent moieties when administered by the oral route.

The selected, parent estrogens encompassed by this invention include 3,17-β-estradiol, estrone, equilin, and 17-α-hydroxydihydroequilenin. Thus, compounds of this invention include 3-hydroxy-17-β-n-butylsulfonoyl estradiol, 3-n-butylsulfonoyl-l7-β-estradiol, 3,17-β-di-n-butylsulfonoyl estradiol, 3-n-butylsulfonoylestrone, 3-n-butylsulfonoyl equilin, 3-n-butylsulfonoyl-17-α-dihyroequilenin, and 3,17-α-di-n-butylsulfonoyl equilenin. These compounds are prepared from the commercially available, parent estrogen, such as 3,l7-β-estradiol, estrone, equilin, and 3,17-α-dihydroxyequilenin, by methods well known in the art (see, e.g., EP-94301871.3).

Generally, the desired parent hydroxy estrogen is sulfonylated with commercially available n-butylsulfonoyl chloride in the presence of a base such as triethylamine in a suitable solvent or mixture of solvents such as tetrahydrofuran (THF), N,N-dimethylformamide (DMF), methylene chloride, and the like. This reaction may be carried out at a variety of temperatures, but ambient temperature is usually sufficient. The crude reaction product may be purified by chromatographic techniques, including, for example, silica gel, and/or may be crystallized from suitable solvents such as mixtures of hexane and diethyl ether. Those parent estrogens which contain two hydroxyl functions may preferentially be sulfonylated on a particular hydroxyl moiety by techniques known in the art. For example, 3-n-butylsulfonyl-17-β-hydroxyestradiol is prepared by using one equivalent of sulfonylating reagent, which will preferentially react at the 3-position. Furthermore, the 3-hydroxy-17-β-n-butylsulfonoyl estradiol compound is prepared by reacting the 3-hydroxyl moiety with an acid sensitive moiety such as acetyl, then sulfonylating the 17-β-hydroxyl group. This differentially modified molecule can then be treated with aqueous acid to remove the acetyl group, leaving the 17-sulfonoylated derivative. Other well known differentially modified derivatives may be used to prepare other desired mono-sulfonated derivatives.

The following examples for the preparation of the compounds of formula I are presented for purposes of illustration and are not be construed as limiting in any way.

### Example 1

### 3,17-β-di-n-butylsulfonoyl estradiol

A solution was prepared of 2.72 g (10 mmol) 3,17-β-estradiol in 100 mL of anhydrous THF. To this solution was added 3.5 g (35 mmol) of triethylamine and 10 mg of dimethylamino pyridine (DMAP). Another solution of 4.7 g (30 mmol) of n-butylsulfonoyl chloride dissolved in 50 mL of THF was slowly added (over a period of 10 minutes) to the stirring estrogen solution. The reaction was allowed to proceed for six days at ambient temperature and under an atmosphere of nitrogen. The reaction mixture was evaporated to dryness *in vacuo.* The resulting residue was suspended in 200 mL of ethyl acetate and extracted with 100 mL of water, 100 mL of 1N hydrochloric acid, 100 mL of 1N sodium hydroxide, and finally again with 100 mL of water. The ethyl acetate solution was dried by filtration through anhydrous sodium sulfate and evaporated to a dark oil, which solidified on standing. This crude product was chromatographed on a silica gel column (HPLC) eluted with a linear gradient of hexane-ethyl acetate (5:1) to hexane-ethyl acetate (2:1). The appropriate fractions were determined by thin layer chromatography, combine, and evaporated to dryness. This yielded 1.77 g of the title compound as a tan amorphous powder.
PMR: Consistent with the proposed structure
MS: m/e=512 (M+) FD
EA: Calc: C, 60.91; H, 7.86 Found: C, 61.16; H, 7.97 C₂₆H₄₀O₆S₂

### Example 2

### 3-n-butylsulfonoyl estrone

A solution was prepared of 2.7 g (10 mmol) of estrone in 100 mL of anhydrous THF. To this solution was added 2 g (20 mmol) of triethylamine and 10 mg of DMAP. Another solution was prepared of 2.5 g (16 mmol) of n-butylsulfonoyl chloride in 25 mL of THF and this was slowly added to the stirring estrone solution. The reaction was allowed to proceed for six days at ambient temperature under a nitrogen atmosphere. The reaction mixture was evaporated to dryness and resuspended to 200 mL of ethyl acetate. The ethyl acetate suspension was extracted with 75 mL of water, 50 mL of 1 N sodium hydroxide, 50 mL of 1 N hydrochloric acid, and 100 mL of water. The resulting solution was dried by filtration through anhydrous sodium sulfate and evaporated to dryness. The product was crystallized from a ether-hexane solution. This yielded 510 mg of the title compound as white powder.
PMR: Consistent with the proposed structure
MS: m/e=390 (M+) FD
EA: Calc: C, 67.66; H, 7.74 Found: C, 67.68; H, 7.83 C₂₂H₃₀O₄S

### Example 3

### 3-n-butylsulfonoyl equilin

A solution was prepared of 300 mg (1.12 mmol) of equilin in 100 mL of THF and 200 mg (2 mmol) of triethylamine was added. Another solution of 200 mg (1.3 mmol) of n-butylsufonoyl chloride in 10 mL of THF was slowly added to the stirring equilin solution. The reaction was allowed to proceed for two days at ambient temperature and under a atmosphere of nitrogen. The reaction mixture was evaporated to dryness and resuspended in 50 mL of ethyl acetate. The ethyl acetate solution was extracted 10 mL of 1 N sodium hydroxide, 10 mL of 1 N hydrochloric acid, and 20 mL of water, then dried with sodium sulfate and evaporated to dryness. The product was crystallized from ether-hexane. This yielded 300 mg of the title compound as a white powder.
PMR: Consistent with the proposed structure
MS: m/e=388 (M+) FD
EA: Calc: C, 68.01; H, 7.26 Found: C, 68.14; H, 7.17 C₂₂H₂₈O₄S

Preferred embodiments of this invention are 3-n-butylsulfonoyl estrone and 3,17-β-di-n-butylsulfonoyl estradiol.

### Test Procedure

To demonstrate the unexpected enhancement of oral absorption of formula I compounds compared to their respective parent compounds, and to illustrate the distinct nature of the n-butyl sulfonate moiety versus commonly used sulfonates, a well known ovariectomized rat model was used (see, e.g., Black, L.J., et al., J. Clin. Invest., 93:63-69, (1994), and U.S. Pat. No. 5,393,763. In this ovex rat model, the effects of an estrogenic or antiestrogenic agent can be measured on several parameters including, for example, uterine weight, serum lipids, and bone loss. In the data presented below, increases in uterine weight illustrate superior estrogenic activity.

Seventy-five day old female Sprague Dawley rats (weight range of 225-275 g) were obtained from Charles River Laboratories (Portage, MI). The animals were either bilaterally ovariectomized or exposed to a sham surgical procedure at Charles River Laboratories, and then shipped after one week. They were housed in groups of three and had *ad libitum* access to food and water. Room temperature was maintained at 22.2° C with 40% relative humidity. The photo-period was 12 hours of light and 12 hours of darkness.

One week after arrival (thus, 14 days post-ovariectomy), the rats were treated with vehicle, 17-α-ethinyl estradiol, parent hydroxy estrogens, or a compound of formula I was initiated. After four days of treatment, blood was collected by cardiac puncture, the animals were sacrificed by carbon dioxide asphyxiation and the uteri were removed and wet weight determined. Uterine weight was routinely reduced about 75% in response to the ovariectomy.

All compounds were given by oral dosage (gavage in 0.5 mL of 1% carboxymethylcellulose suspension). For comparison purposes, the data represented are for 0.1 mg/kg for all compounds. The positive control was 0.1 mg/kg of 17-α-ethinyl estradiol, a known, orally available estrogen.

**Table 1**

| | Uterine weight as percent control @ 0.1 |
|---|---|
| Compound | mg/kg P.O. |
| 17-β-estradiol | 43 |
| 3,17-β-di-methanesulfonoyl estradiol | 0 |
| 3-methanesulfonoyl-17-β-estradiol | 17 |
| 3-phenylsulfonoyl-17-β-estradiol | 0 |
| 3,17-β-di-n-butylsulfonoyl estradiol | 112 |
| Estrone | 35 |
| 3-methanesulfonoyl estrone | 0 |
| 3-phenylsulfonoyl estrone | 10 |
| 3-n-butylsulfonyl estrone | 67 |
| Equilin | 47 |
| 3-n-butylsulfonoyl equilin | 119 |

In each comparison, the n-butylsulfonoyl estrogens were surprisingly more potent than their hydroxyparent estrogens and the n-butylsulfonoyl derivatives were uniquely more potent than the commonly used "chemical-protecting" sulfonates.

Accordingly, the present invention also provides methods for treating estrogen deficient pathologies including, for example, lack of birth control, post-menopausal syndrome including, for example, osteoporosis, cardiovascular disease, restenosis, and hyperlipidemia, certain cancers in men such as prostate cancer, acne, hirsutism, dysfunctional uterine bleeding, dysmenorrhea, and atrophic vaginitis comprising administering to a mammal in need of such treatment an effective amount of a compound of formula I, and, optionally, an effective amount of a progestin. One of ordinary skill in the art will recognize that estrogenic agents, particularly the highly soluble and orally available compounds of the present invention, have a multitude of applications for treating estrogen deficient pathologies well beyond those listed *infra.* The present invention, therefore, contemplates and encompasses such maladies although not specified by name.

As used herein, the term "treatment" or a derivative thereof, is defined to include its generally accepted meaning which includes, for example, prophylactically protecting a subject from incurring the symptoms of an estrogen deficient pathology, holding in check such symptoms, and/or ameliorating existing symptoms. As such, the present method includes both medical therapeutic and/or prophylactic treatment, as appropriate. As used herein, the term "progestin" includes compounds having progestational activity such as, for example, progesterone, norethylnodrel, nogestrel, megestrol acetate, norethindrone, and the like.

The method of administration of each progestin-based agent is consistent with that which is known in the art. For the majority of the methods of the present invention, compounds of formula I are administered continuously from 1 to 3 times daily. However, cyclical therapy may especially be useful, particularly when used for birth control. In the case of restenosis, therapy may be limited to short (1-6 month) intervals following medical procedures such as angioplasty.

As used herein, the term "effective amount" means an amount of compound of the present invention which is capable of treating the symptoms of the various pathological conditions herein described. The specific dose of a compound administered according to this invention will, of course, be determined by the particular circumstances surrounding the case including, for example, the compound administered, the route of administration, the state of being of the patient, and the pathological condition being treated. A typical daily dose of a formula I compound contains a nontoxic dosage level of from about 0.01 mg to about 5.0 mg/day when used concurrently or sequentially with progestin, which is administered at a dose from about 0.1 mg to about 5.0 mg/day. When administered without progestin, compounds of formula I are administered at a dose from about 0.1 mg to about 10 mg/day. Preferred daily doses generally are from about 1.0 mg to about 5.0 mg/day.

The compounds of the present invention can be administered by a variety of routes including oral, rectal, transdermal, subcutaneous, intravenous, intramuscular, and intranasal. These compounds preferably are formulated prior to administration, the selection of which will be decided by the attending physician. Thus, another aspect of the present invention is a pharmaceutical composition comprising an effective amount of a compound of formula I, optionally containing an effective amount of progestin, and a pharmaceutically acceptable carrier, diluent, or excipient.

The total active ingredients in such formulations comprises from 0.1% to 99.9% by weight of the formulation. By "pharmaceutically acceptable" it is meant the carrier, diluent, and excipients, must be compatible with the other ingredients of the formulation, and not deleterious to the recipient thereof.

Pharmaceutical formulations of the present invention are prepared by procedures known in the art using well known and readily available ingredients. For example, the compounds of formula I, with or without a progestin compound, are formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl-pyrrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonite; lubricants such as talc, calcium and magnesium stearate, and solid polyethyl glycols.

Compounds of the present invention also can be formulated as elixirs or solutions for convenient oral administration or as solutions appropriate for parenteral administration, for example, by intramuscular, subcutaneous or intravenous routes. Additionally, the compounds are well suited to formulation as sustained release dosage forms and the like. The formulations can be so constituted that they release the active ingredient only or preferably in a particular physiological location, possibly over a period of time. The coatings, envelopes, and protective matrices may be made, for example, from polymeric substances or waxes.

Compounds of formula I, alone or in combination with a progestin, generally will be administered in a convenient formulation. The following formulation examples only are illustrative and are not intended to limit the scope of the present invention.

### Formulations

In the formulations which follow, "active ingredient" means a compound of formula I, or a solvate thereof.

### Formulation 1: Gelatin Capsules

Hard gelatin capsules are prepared using the following:

| Ingredient | Quantity (mg/capsule) |
|---|---|
| Active ingredient | 0.01 - 10 |
| Starch, NF | 0 - 650 |
| Starch flowable powder | 0 - 650 |
| Silicone fluid 350 centistokes | 0 - 15 |

The formulation above may be changed in compliance with the reasonable variations provided.

A tablet formulation is prepared using the ingredients below:

### Formulation 2: Tablets

| Ingredient | Quantity (mg/tablet) |
|---|---|
| Active ingredient | 0.01 - 10 |
| Cellulose, microcrystalline | 200 - 650 |
| Silicon dioxide, fumed | 10 - 650 |
| Stearate acid | 5 - 15 |

The components are blended and compressed to form tablets.

Alternatively, tablets each containing 0.01 - 10 mg of active ingredient are made up as follows:

### Formulation 3: Tablets

| Ingredient | Quantity (mg/tablet) |
|---|---|
| Active ingredient | 0.01 - 10 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60° C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.01 - 10 mg of medicament per 5 ml dose are made as follows:

### Formulation 4: Suspensions

| Ingredient | Quantity (mg/5 ml) |
|---|---|
| Active ingredient | 0.01 - 10 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The medicament is passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.

An aerosol solution is prepared containing the following ingredients:

### Formulation 5: Aerosol

| Ingredient | Quantity (% by weight) |
|---|---|
| Active ingredient | 0.25 |
| Ethanol | 25.75 |
| Propellant 22 (Chlorodifluoromethane) | 70.00 |

The active ingredient is mixed with ethanol and the mixture added to a portion of the propellant 22, cooled to 30° C, and transferred to a filling device. The required amount is then fed to a stainless steel container and diluted with the remaining propellant. The valve units are then fitted to the container.

Suppositories are prepared as follows:

### Formulation 6: Suppositories

| Ingredient | Quantity (mg/suppository) |
|---|---|
| Active ingredient | 10 |
| Saturated fatty acid glycerides | 2,000 |

The active ingredient is passed through a No. 60 mesh U.S. sieve and suspended in the saturated fatty acid glycerides previously melted using the minimal necessary heat. The mixture is then poured into a suppository mold of nominal 2 g capacity and allowed to cool.

An intravenous formulation is prepared as follows:

### Formulation 7: Intravenous Solution

| Ingredient | Quantity |
|---|---|
| Active ingredient | 50 mg |
| Isotonic saline | 1,000 mL |

The solution of the above ingredients is intravenously administered to a patient at a rate of about 1 mL per minute.

### Formulation 8: Combination Capsule I

| Ingredient | Quantity (mg/capsule) |
|---|---|
| Active ingredient | 0.1 - 1 |
| Norethylnodrel | 0.01 - 2 |
| Avicel pH 101 | 50 |
| Starch 1500 | 117.50 |
| Silicon Oil | 2 |
| Tween 80 | 0.50 |
| Cab-O-Sil | 0.25 |

### Formulation 9: Combination Capsule II

| Ingredient | Quantity (mg/capsule) |
|---|---|
| Active ingredient | 0.1 - 1 |
| Norethylnodrel | 0.01 - 2 |
| Avicel pH 101 | 82.50 |
| Starch 1500 | 90 |
| Silicon Oil | 2 |
| Tween 80 | 0.50 |

### Formulation 10: Combination Tablet

| Ingredient | Quantity (mg/capsule) |
|---|---|
| Active ingredient | 0.1 - 1 |
| Norethylnodrel | 0.01 - 2 |
| Corn Starch NF | 50 |
| Povidone, K29-32 | 6 |
| Avicel pH 101 | 41.50 |
| Avicel pH 102 | 136.50 |
| Crospovidone XL10 | 2.50 |
| Magnesium Stearate | 0.50 |
| Cab-O-Sil | 0.50 |

## Claims

1. A compound of formula I 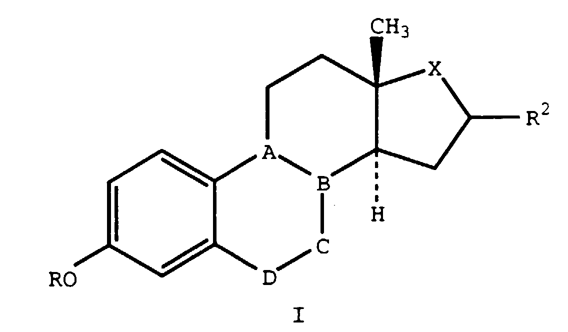 wherein
X is α-CH₂OR¹ when A is α-hydro, A and B together are ethylene, and C and D together are ethylene, β-CH₂OR¹, or -CO-;
R is -H or n-butylsulfonoyl;
R¹ is R provided that at least one of R and R¹ must be n-butylsulfonoyl;
R² is -H, α-or β-hydroxy, or α-or β-n-butylsulfonoyl;
A is 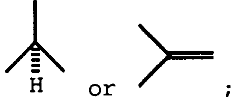
B is 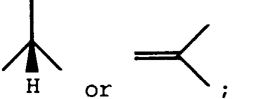
C is -CH₂ or 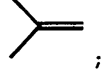 and
D is -CH₂ or

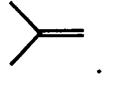

2. A compound according to Claim 1 which is 3-hydroxy-17-β-n-butylsulfonoyl estradiol, n-butylsulfonoyl-17-β-estradiol, 3,17-β-di-n-butylsulfonoyl estradiol, 3-n-butylsulfonoyl estrone, 3-n-butylsulfonoyl equilin, 3-hydroxy-17-α-n-butylsulfonoyl dihydroequilenin, 3-n-butylsulfonoyl-17-α-dihydroequilenin, or 3,17-α-di-n-butylsulfonyl equilin.

3. The use of a compound of Claim 1 for preparation of a medicament useful for treating estrogen deficient pathologies.

4. A pharmaceutical formulation comprising a compound according to any one of Claims 1 or 2, and, optionally, progestin, in combination with a pharmaceutically acceptable carrier, diluent, or excipient.
